Europäisches Patentamt

European Patent Office

Office européen des brevets

(11) Publication number: **0 353 772**
**A2**

# EUROPEAN PATENT APPLICATION

(21) Application number: 89114458.6

(51) Int. Cl.⁴ **A61K 37/02**

(22) Date of filing: 04.08.89

(30) Priority: 05.08.88 US 229133

(43) Date of publication of application:
07.02.90 Bulletin 90/06

(84) Designated Contracting States:
AT BE CH DE ES FR GB GR IT LI LU NL SE

(71) Applicant: **ONCOGEN LIMITED PARTNERSHIP**
**3005 First Avenue**
**Seattle, WA 98121(US)**

(72) Inventor: **Twardzik, Daniel R.**
**9600 Timberlane Place**
**Bainbridge Island, WA 98112(US)**
Inventor: **Purchio, Anthony F.**
**801 33rd Avenue East**
**Seattle Wash. 98112(US)**
Inventor: **Ranchalis, Jane E.**
**2142 8th Avenue West**
**Seattle Wash. 98119(US)**
Inventor: **Stevens, Victor**
**298 Woodward Avenue**
**Buffalo, N.Y. 14214(US)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) Psoriasis treatment with TGF-beta.

(57) Methods are provided for inhibiting growth of cells, particularly epidermal cells. A means for inhibiting proliferation due to stimulation by mitogens, including cytokines such as interleukin I (IL-1) is also provided as is a means for inhibiting or suppressing the functionability of infiltrating immune cells producing cytokines. The method may be used as a treatment for hyperplastic dermatological disorders such as psoriasis.

EP 0 353 772 A2

# PSORIASIS TREATMENT WITH TGF-$\beta$

## INTRODUCTION

### Technical Field

This invention relates to methods for inhibiting proliferation of epidermal cells in particular keratinocytes, as a means for treating hyperplastic dermatologic disorders.

### Background

Psoriasis is a chronic cutaneous disease characterized by an increase in the rate of proliferation of the epidermis. The major cell type in the hyperproliferating epidermis is the keratinocyte. Keratinocytes are stimulated to grow by transforming growth factor TGF-$\alpha$. Exposure of cultured keratinocytes to exogenous TGF-$\alpha$ results in increased levels of messenger RNA for endogenous TGF-$\alpha$. Thus the growth factor cycle in keratinocytes is of an auto stimulatory type.

The underlying dermis in psoriatic skin specimens contains an infiltrate of cells of the immune type, predominantly lymphocytes and macrophages. Factors elaborated by these immune cells also stimulate proliferation of the epidermis. Stimulatory factors produced by the immune cells include cytokines such as interleukin-1 and interleukin-2.

The family of peptides known as transforming growth factor type $\beta$ (TGF-$\beta$) regulate cell growth and differentiation. These growth regulatory polypeptides can both stimulate and inhibit cell proliferation, depending largely on the cell type. TGF-$\beta$1 and TGF-$\beta$2 both have a potent anti-proliferative effect on many normal epithelial cells. Furthermore, TGF-$\beta$ also modulates Class II antigen expression and thus can suppress inflammatory reactions. In addition TGF-$\beta$ is a potent modulator of immune function and can inhibit lymphocyte blastogenesis, antibody secretion by $\beta$ cells and interleuleukin and other related cytokine-induced phenomena.

Keratinocytes are of epithelial origin. There is therefore an interest in determining whether the hyperplasia found in acanthosis categorized skin diseases may be inhibited by these anti-proliferative factors. Furthermore, it is of interest to determine whether TGF-$\beta$ will further alleviate psoriatic symptoms by suppression of immune reactions.

### Relevant literature

A review of the family of compounds under the designation TGF-$\beta$ is provided by Sporn et al., Science (1986) 233:532-534. The structure and functional relationship of CIF-$\beta$ (cartilage-inducing factor B) to TGF-$\beta$ is demonstrated by Seyedin et al., J. Biol. Chem. (1987) 262:1946-1949. See also Seyedin et al., Proc. Natl. Acad. Sci. USA (1985) 82:2267-2271.

## SUMMARY OF THE INVENTION

Novel methods and compositions comprising at least one transforming growth factor $\beta$ or congeners thereof are provided for treating cutaneous diseases characterized by an increased rate of proliferation of the epidermis. The compositions are used to inhibit the hyperplastic conditions that result from mitogen, including cytokinin, stimulated proliferation of the epidermis. The compositions may be applied topically to affected skin, preferably in a formulation providing for enhanced absorption through the stratum corneum.

## BRIEF DESCRIPTION OF THE DRAWINGS

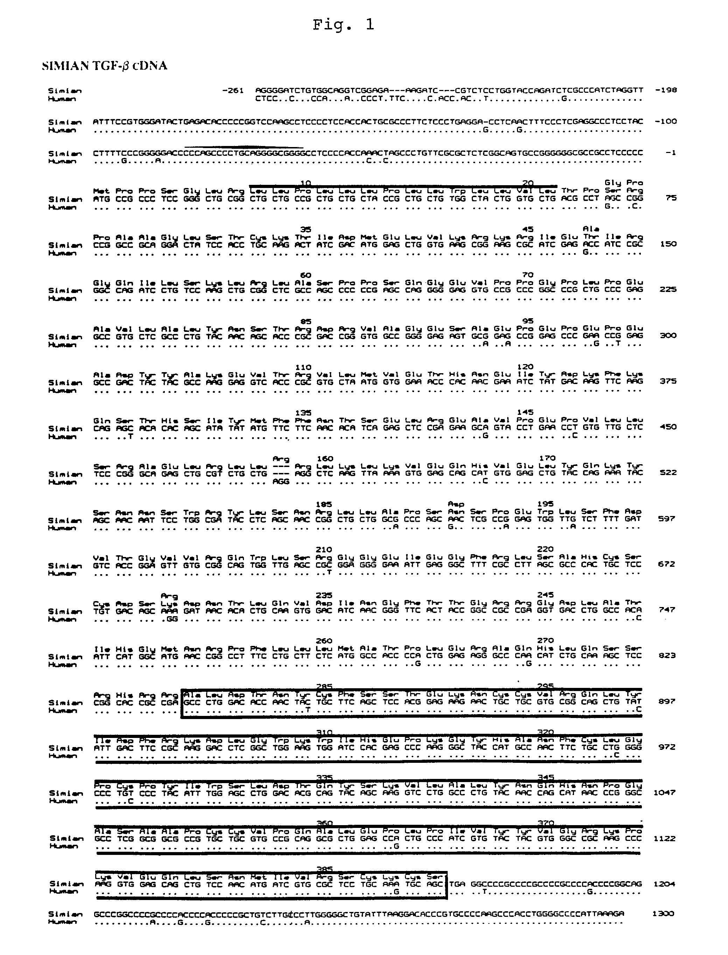

Figure 1 shows nucleotide sequence of simian TGF-β cDMA and deduced amino acid sequence. The 1600bp insert of pTGF-β was subcloned into the M13mpl8 and M13mpl9 cloning vectors (Yanisch-Perron et. al, 1985) and sequenced on both strands using the dideoxy chain-termination method (Sanger et. al., 1977). The human and simian DNA sequences are identical (dots) except where indicated; amino acid differences between the human and simian proteins are shown in the top line. The mature TGF-β sequence is boxed and the signal peptide is overlined. A palindrome in the 5′ untranslated region is also overlined.

Figure 2 shows a Franz diffusion chamber.

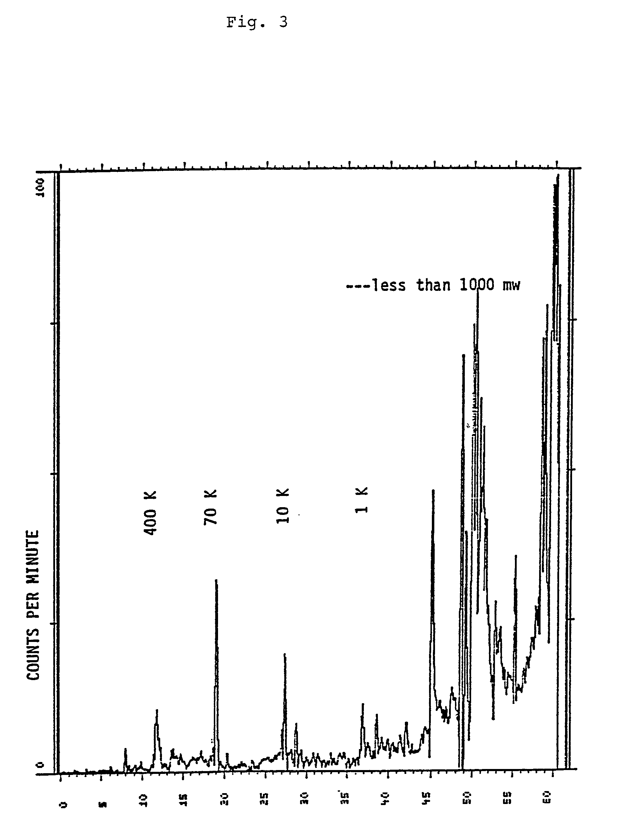

Figure 3 shows an HPLC chromatograph of [$^{35}$S] recovered from the pooled receptor solutions of all chambers that received topical TGF-β. Separation was accomplished on a gel-permeation column using PBS as the solvent. Approximate molecular weights of peaks are noted based upon from previously injected standards.

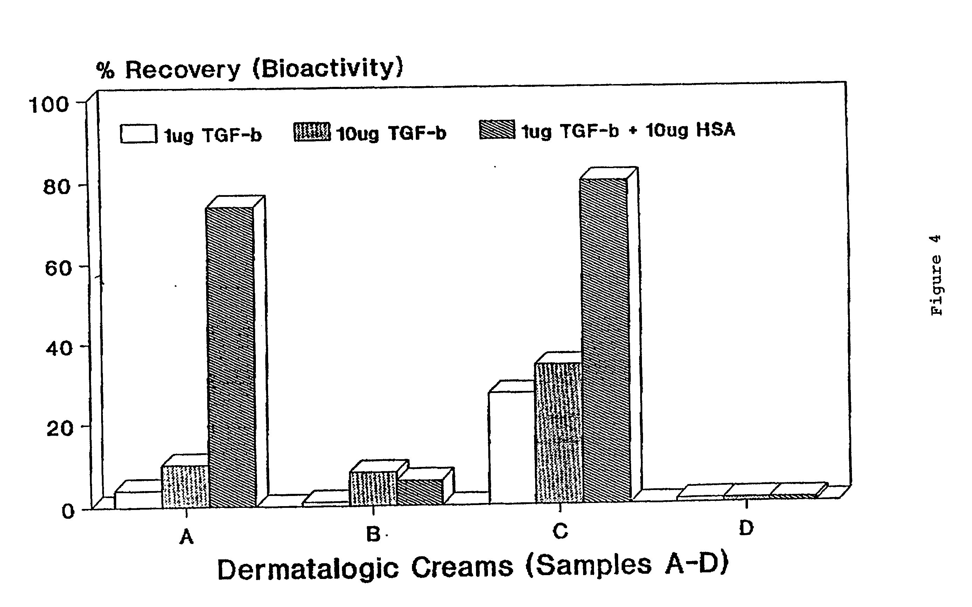

Figure 4 shows the percentage recovery of TGF-β from four dematologic cream bases.

## DESCRIPTION OF THE SPECIFIC EMBODIMENTS

In accordance with the subject invention, methods and compositions are provided for inhibiting mitogen-stimulated growth of keratinocytes in the epidermis for treatment of psoriasis and related dermatological conditions. The compositions comprise polypeptides having substantially the same amino acid sequence as at least a portion of a consecutive amino acid sequence of TGF-β. Keratinocyte mutogens which can be counteracted include EGF and TGF-β.

The nucleotide sequence of simian TGF-β DNA and the human and simian amino acid sequences of TGF-β are provided in Figure 1. Mature TGF-β1 has the following sequence:

```
                5          10         15         20
A-L-D-T-N-Y-C-F-S-S-T-E-K-N-C-C-V-R-Q-L-
                25         30         35         40
Y-I-D-F-R-K-D-L-G-W-K-W-I-H-E-P-K-G-Y-H-
                45         50         55         60
A-N-F-C-L-G-P-C-P-Y-I-W-S-L-D-T-Q-Y-S-K-
                65         70         75         80
V-L-A-L-Y-N-Q-H-N-P-G-A-S-A-A-P-C-C-V-P-
                85         90         90        100
Q-A-L-E-P-L-P-I-V-Y-Y-V-G-R-K-P-K-V-E-Q-
                105        110
L-S-N-M-I-V-R-S-C-K-C-S
```

and mature TGF-β2 has the following sequence:

$$5 \qquad 10 \qquad 15 \qquad 20$$

A-L-D-A-A-Y-C-F-R-N-V-Q-D-N-C-C-L-R-P-L-

$$25 \qquad 30 \qquad 35 \qquad 40$$

Y-I-D-F-K-R-D-L-G-W-K-W-I-H-E-P-K-G-Y-N-

$$45 \qquad 50 \qquad 55 \qquad 60$$

A-N-F-C-A-G-A-C-P-Y-L-W-S-S-D-T-Q-H-S-R-

$$65 \qquad 70 \qquad 75 \qquad 80$$

V-L-S-L-Y-N-T-I-N-P-E-A-S-A-S-P-C-C-V-S-

$$85 \qquad 90 \qquad 95 \qquad 100$$

Q-D-L-E-P-L-T-I-L-Y-Y-I-G-K-T-P-K-I-E-Q-

$$105 \qquad 110$$

L-S-N-M-I-V-K-S-C-K-C-S

The one-letter designations for the various amino acids are as follows: A = alanine; R = arginine; N = asparagine; D = aspartic acid; C = cysteine; Q = glutamine; E = glutamic acid; G = glycine; H = histidine; I = isoleucine; L = leucine; K = lysine; M = methionine; F =phenylalanine; P = proline; S = serine; T = threonine; W = tryptophan; Y = tyrosine; and V = valine.

The sequence of the compositions of interest will usually be comparable to a sequence of a segment of the TGF-$\beta$ molecule, including TGF-$\beta$ precursor polypeptides. The compositions will include a sequence corresponding substantially to the portion of the molecule responsible for the observed biological effect of TGF-$\beta$ for inhibiting proliferation of epidermal cells, in particular, keratinocytes.

The compositions of this invention will have as the active moiety at least one TGF-$\beta$ of at least about 8 amino acids, usually at least about 25 amino acids, more usually at least about 35 amino acids, up to the full length of a TGF-$\beta$ or TGF-$\beta$ precursor polypeptides. Preferably, the composition comprises homodimers of TGF-$\beta$. By homodimer is intended a polypeptide comprising two idential amino-acid chains of at least 8 amino acids each. A preferred method of linkage of the two chains is via an interchain disulfide bond, where each chain includes at least one cysteine, although other linkages are possible.

It will be appreciated that the amino-acid sequence need not correspond exactly to the sequences given above, but may be modified by from 1 to 4 conservative or non-conservative substitutions, including deletions and insertions usually involving not more than about 1 amino acid, where the modifications may include D-amino acids, without significantly affecting the activity of the product. Therefore the subject polypeptides may be subject to various changes, such as insertions, deletions, and substitutions, either conservative or non-conservative, where such changes might provide for certain advantages in their use. By conservative substitutions is intended combinations such as G, A; V, I, L; D, E; N, Q; F, T; K, R; and S, Y, W.

TGF-$\beta$ and congeners thereof may be obtained in a variety of ways. They are available from naturally occurring sources, such as bovine bone and human platelets. Methods for their preparation include the following: The peptides may be synthesized on a solid support using various automated synthesizers which are commercially available in accordance with known protocols. See, for example, Stewart and Young, Solid Phase Peptide Synthesis, 2nd ed., Pierce Chemical Company (1984); and Tam et al., J. Am. Chem. Soc. (1983) 105:6442. Additionally, they may be prepared using recombinant DNA techniques. Thus they may be obtained from growth medium conditioned by Chinese hamster ovary cells (CHO) containing the Simian gene for TGF-$\beta$ expressed at high levels by dihydrofalate reductase (dhfr) gene amplification. Methods for obtaining TGF-$\beta$ precursor polypeptides and homodimers from transformed CHO cells are described in Gentry et. al. Molecular and Cellular Biology (1987) 7:3417-3427 and U.S. Patent Application Serial Number 147,842 filed January 25, 1988, which disclosures are incorporated herein by reference. The TGF-$\beta$ or fragments thereof also can be prepared by employing recombinant techniques as described in copending U.S. application application Serial No. 148,267 filed January 25, 1988 which disclosure is incorporated herein by reference.

The TGF-$\beta$ can be purified so as to be substantially free of cellular components employing various purification techniques. These techniques can include solvent extraction, gel permeation chromatography, reversed-phase HPLC, electrophoresis, ion or exchange, affinity chromatography or the like.

For use in culture, the transforming growth factor may be derived from any vertebrate source, not necessarily the same source as the cells. However, for treatment of a mammal, it will be desirable to employ a component with substantially homologous to the naturally occurring component so as to minimize the potential for an immunogenic response. Usually the component will differ by fewer by than 5 mole percent, more usually by fewer than 2 mole percent, from the amino acid sequence of the naturally occurring sequence from the host.

The TGF-$\beta$ or congeners thereof are generally formulated into a cream or ointment base for application to affected skin. The TGF-$\beta$ or congers thereof may be used as one compound. The compound is provided usually at at least about 50 percent purity and up to complete purity. The polypeptide will be present in an in the treatment composition in an epithelial cell proliferation inhibiting amount; generally greater than 50% inhibition of cultured keratinocytes can be achieved with about 20 to about 40 ng/ml of mature TGF-$\beta$. The most effective concentration of TGF-$\beta$ for inhibiting proliferation of the keratinocytes may be determined by adding various concentrations of TGF-$\beta$ to cultured keratinocytes and monitoring the amount of inhibition of cell proliferation. The amount required to obtain inhibition will vary depending upon the relative potency of the TGF-$\beta$ used, for example, increased concentrations would be used for TGF-$\beta$ congeners having a lower relative bioactivity than mature TGF-$\beta$. For mature TGF-$\beta$, the concentration used is generally greater than about 20ng/ml.

If desired, TGF-$\beta$-containing compositions can be incorporated in bandages and other dressings to provide for continuous exposure of the affected area to the peptide. The nature of the carriers may vary widely, and will depend on the intended location and type of application. Examples of other applications include injection and aerosol applications and pressurized administration as provided by a dermajet.

To preserve the biological activity of the TGF-$\beta$, the TGF-$\beta$ can be formulated into a cream vehicle such as an oil and water (o/w) type, creamy, white emulsion base comprising nonionic emulsified agents, for example, glycerol stearate, PEG-40 stearate, sorbitan stearate in a concentration of about 0.5-15%; emollients, for example fatty acid esters, of about 1-20%; humectants, for example sorbitol, about 1-15%; and preservatives, for example benzyl alchohol or kathon C.G., about 0.1-2%. A preferred formulation also includes a protein carrier, such as human serum albumin, collagen and derivatives thereof, about 0.01 to 0.1 g/g of cream base to aid in stabilization of the TGF-$\beta$. The percent recovery of bioactive TGF-$\beta$ from such formulations is higher than that from formulations wherein the emollients include dimethicone, petrolatum or the alike, or humectants such as glycerin.

The subject peptides may be used either alone or with at least one other anti-proliferative compound, for example tumor necrosis factor-$\beta$, tumor necrosis factor-$\alpha$, TGF-$\beta$, or platelet factor 4, and the like.

Use of TGF-$\beta$ and Congeners

The subject compositions find use in the treatment of hyperplasia associated with acanthosis-categorized skin diseases and in alleviating psoriatic symptoms associated with cytokine-induced phenomena.

Besides being used for their growth inhibiting properties, TGF-$\beta$ and fragments thereof may be used in diagnostics. Thus, TGF-$\beta$ or fragments thereof may be joined to a label, such as a radioisotope, enzyme, fluorescer, chemiluminescer, enzyme fragment, particle, etc. Examples of diagnostic uses for TGF-$\beta$ include titrating the number of receptors for TGF-$\beta$ on a cell. Identification of receptors for TGF-$\beta$ is an indication of potential responsiveness of the cell to the growth inhibitor effects of TGF-$\beta$. Thus a biopsy specimen may be assayed for the presence of receptors for TGF-$\beta$. Patients lacking such receptors are unlikely to respond to treatment with the subject compositions. Furthermore, by quantitating the number of receptors following different treatment regimens, it may be possible to optimize the treatment protocol, for example time between treatments, or sequence and/or formulation of particular treatment compositions used. For example, those treatment regimens which increase the number of receptors for TGF-$\beta$ could be expected to result in an improved response to compositions comprising TGF-$\beta$.

The following examples are offered by way of illustration and not by way of limitation.

EXPERIMENTAL

TGF-$\beta$: Unless otherwise indicated, the TGF-$\beta$ used in all experiments is TGF-$\beta$1 (homodimer) obtained from bovine bone as described by Seyedin et al. with an approximate apparent molecular weight of 24,000.

## Example 1

Inhibition of DNA Synthesis in Keratinocytes by TGF-$\beta$1 Alone or in Combination with EGF or TGF-$\alpha$

Human keratinocytes were harvested from neonatal foreskins by trypsinization. The skin was washed in 0.9% saline with penicillin and streptomycin and placed in an 0.2% trypsin solution for 12 hrs at room temperature. The upper layers of the epidermis were removed and the foreskins were placed in McCoy's 5A medium. Basal keratinocytes were harvested by brushing the surface of the foreskins with curved forceps. The cells were washed in medium, counted and plated at 40,000 cells/cm².

Table 1

| TGF-$\beta$1 Inhibition of Growth of Human Keratinocytes in Culture | | | | | | |
|---|---|---|---|---|---|---|
| ng/ml TGF-$\beta$1 | TGF-$\beta$1 Only | | + 10 ng/ml EGF | | + 10 ng/ml TGF-$\alpha$ | |
| | DPM 3H-Tdr $\mu$g DNA | % Control | DPM 3H-Tdr $\mu$g DNA | % Control | DPM 3H-Tdr $\mu$g DNA | % Control |
| Control | 7037 ± 461 | -- | 12612 ± 935 | -- | 8705 ± 368 | -- |
| 40 | 810 ± 105* | 12 | 1491 ± 406* | 12 | 1540 ± 312* | 18 |
| 4 | 4490 ± 366* | 64 | 4668 ± 668* | 37 | 7169 ± 740* | 82 |
| 0.4 | 6789 ± 426 | 96 | 8157 ± 851* | 65 | 8532 ± 427* | 96 |

* Significantly different from control (N = S)

After incubation at 37°C, 5% $CO_2$/95% air for 12 to 18 hrs to allow cells to attach, the medium was replaced with medium containing TGF-$\beta$ alone or with medium containing 10 ng/ml TGF-$\alpha$ or EGF in combination with TGF-$\beta$. Control cultures received medium alone. After six days, the cells were pulsed with 1 $\mu$Ci/ml tritiated thymidine for 6 hrs. The cultures were then harvested, total DNA was measured and the tritiated thymidine incorporated into DNA was determined. The specific activity of the DNA in test cultures was compared with that of control cultures. As shown in Table 1, TGF-$\beta$ (0.4 to 40 ng/ml) either alone or in combination with either EGF (10 ng/ml) or TGF-$\alpha$ (10 ng/ml) inhibited growth of human keratinocytes in culture. Thus, TGF-$\beta$ inhibited the proliferation of keratinocytes in a dose-dependent manner. It also prevented the stimulatory effect of EGF or TGF-$\alpha$ on the proliferation of the keratinocytes.

## Example 2

Reversal of Interleukin-1 Stimulation of DNA Synthesis in Keratinocytes

Mouse keratinocytes were prepared using the trypsin flotation method. The cells were grown in Dulbecco's Modified Eagles Medium (DMEM) with 10% heat-inactivated calf serum. The following day the medium was changed to fresh serum-free medium (DMEM/modified F-12 medium, 3:2) containing 0.06 mM $Ca^{2+}$ to arrest the keratinocytes in $G_1$ ($G_0$) and to prevent differentiation.

The cells to be tested were cultured in 96-well tissue culture plates (Falcon 302) at a constant ratio of 3 x 10³ cells per 50 $\mu$l of DMEM/modified F-12 medium containing 10% fetal calf serum. Compositions to be tested were in 0.2 M acetic and were lyophilized in sterile 12 x 75 mm tubes (Falcon 2063). Recombinant IL-I was purchased from Collaborative Biochemicals, Lexington, Mass. and was used at the concentrations specified in Table 2. TGF-$\beta$ was obtained from human platelets.

Table 2

| Inhibition of DNA Synthesis in Keratinocytes Stimulated With Interleukin-1 (IL-1) | | | |
|---|---|---|---|
| IL-1 (Half-maximal Units/ml) | % Stimulation DNA Synthesis | Concentration TGF$\beta$ (ng/ml) | % Inhibition DNA Synthesis |
| 30 | 27 | 5 | 12 |
| 90 | 80 | 10 | 41 |
| 120 | 97 | 20 | 58 |

Lyophilized samples were resuspended in DMEM with 10% fetal calf serum and were added in 50 $\mu$l to the test wells, in triplicate, five hours after plating. After incubation at 37°C in a humidified 5% $CO_2$-95% air atmosphere for 72 hours, [$^{125}$I]IdUdr (Amersham IM355), a thymidine analog, was added in 50 $\mu$l of medium (1 $\mu$ci/ml). The cells were incubated for an additional 24 hours, washed once with phosphate-buffered saline (PBS), then fixed for 10 minutes with 200 $\mu$l methanol. The cells were then air-dried for 15 minutes, then solubilized in 200 $\mu$l 1M NaOH for 20 minutes at 65°C. Labeled material was collected using the Titertech Supernatant Collection System (Flow Laboratories, 78-210-05). Inhibition of growth was expressed as the percent decrease of [$^{125}$I]IdUdr incorporation into treated cells as compared to the incorporation into untreated cells. As shown in Table 2, IL-1 (30 to 120 units/ml) stimulated DNA synthesis in keratinocytes; TGF-$\beta$ (5 to 20 ng/ml) not only counteracted the IL-1 induced stimulation of DNA synthesis, but also inhibited DNA synthesis further.

Example 3

Comparison of TGF-$\beta$1 and TGF-$\beta$2 Inhibition of Proliferation of Epidermal Cells

Keratinocytes from neonatal foreskins were cultured as described in Example 1. After the cells had plated out, the medium was replaced with medium containing either EGF (10 ng/ml) or TGF-$\alpha$ (10 mg/ml), and one of three concentrations of TGF-$\beta$2 (see Table 3). After six days of culture with the combination of growth factors, the cultures were pulsed with 3H-thymidine for 6 hrs and the specific activity of the DNA of the cells was determined As shown in Table 3, in the presence of either EGF or TGF-$\alpha$, both of which which stimulate proliferation, both TGF-$\beta$1, or TGF-$\beta$2 were both able to antagonize the proliferation of the cells. The TGF-$\beta$ factors were similar in potency at inhibiting keratinocyte proliferation.

Example 4

Percutaneous Absorption of TGF-$\beta$

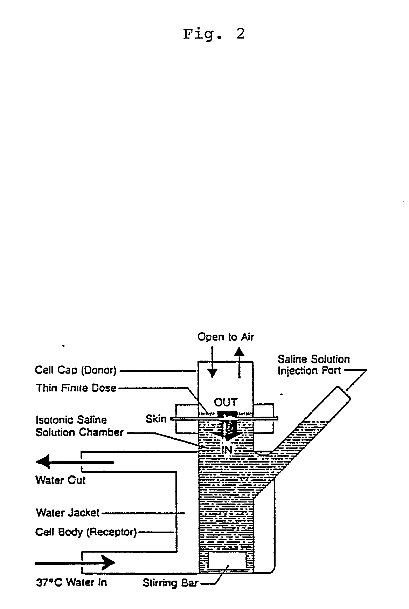

Compounds having an apparent molecular weight of greater than about 2000 kDA do not penetrate well into the skin. The effects of an enhancer (Azone), were therefore evaluated. Percutaneous absorption of TGF-$\beta$ through cadaver human skin was determined in a Franz diffusion chamber (Figure 2) using the finite-dose Franz diffusion model. In use, skin is mounted between the cell cap (donor) and the cell body

Table 3

| INHIBITION OF KERATINOCYTES STIMULATED WITH EGF OR TGF-α | | | | | | | |
|---|---|---|---|---|---|---|---|
| ng/ml TGF-β | EGF (10 ng/ml) | | | | TGF-α (10 mg/ml) | | | |
| | TGF-β1 | | TGF-β2 | | TGF-β1 | | TGF-β2 | |
| | 3H-TdR/μg DNA | % Ctrl | 3H-TdR/μg DNA | % Ctrl | 3H-TdR/μg DNA | % Ctrl | 3H-TdR/μg DNA | % Ctrl |
| Control | 3486±376 | - | 12922±1369 | - | 2306±118 | - | 6331±378 | - |
| 40 | 183±31* | 5 | 1743±327* | 13 | 895±164* | 38 | 1754±166 | 28 |
| 4 | 1931±182* | 55 | 10205±1123 | 79 | 1639±184 | 71 | 6951±501 | 108 |
| 0.4 | 3767±512 | 108 | 13003±802 | 101 | 2702±397 | 117 | 7281±609 | 115 |

* Significantly different from control.

(receptor). The dermis is bathed from below by an isotonic saline solution injected through a port provided for that purpose. Temperature is maintained at 37°C by a thermostatically controlled border which enters the lower port of the water jacket surrounding the saline solution chamber, and circulates out through the upper port. Water is supplied and removed by 2 (upper and lower) stainless-steel manifolds connected to a constant temperature bath. Homogeneous distribution of temperature in the saline bathing solution is accomplished by the agitating motion of a teflon-covered magnetic stirring bar, driven by an external magnet mounted on a timing motor. The cell cap is open to the air, exposing the epidermis to the ambient conditions of the laboratory environment. The open cap allows for finite dose applications of study compounds to the epidermis by use of a micropipette or stirring rod.

For analysis of TGF-β absorption, the method is as follows. Human dermatomed skin, obtained from the back at autopsy and stored at 70°C, was rapidly thawed at room temperature. The epidermis, with attached stratum corneum, was removed from the dermis by sandwiching the skin between two aluminum blocks heated to 60°C for 2 min. The epidermal strip was mounted onto Franz diffusion chambers (Figure 2) having 1 cm² available surface area.

$[^{35}S]$-TGF-β ($3\times10^6$ cpm μg) provided in 30% acetonitrile, 0.05% TFA was dried in a Speed-vac system after the addition of 50 μg of bovine serum albumin. Once dried, the $[^{35}S]$-TGF-β was partitioned into two aliquots, one being dissolved in Vehicle-N (consisting of 4% propylene glycol, 47.5% ethanol and 4% isopropyl alcohol in water and the other portion into Vehicle-N containing 5% Azone (dodecylazacycloheptane), a penetration enhancer, in water. Azone is prepared by Nelston Laboratories. Twenty microliters of each vehicle mix were pipetted to separate duplicate skin samples. Ten μl of each vehicle were assayed as standards.

The chamber solution was removed for assay and replaced with fresh solution at 4, 8, 12, 24, 36, and 48 hrs after TGF-β application. 3 ml of the chamber solution was mixed with 10 ml scintillation fluid and counted in a scintillation counter. The remaining chamber solution from each sample (approx. 2 ml) was pooled with the other chamber solution samples and stored at -30°C.

Table 4

| Rate of Isotope Penetration and Total Isotope Distribution from the Topical Application of [$^{35}$S]-TGF-$\beta$ to Human Skin | | | |
|---|---|---|---|
| Chamber Solution | Vehicle-N % Dose/Hr | Vehicle-N + Azone %Dose/Hr | Time (Hrs) |
| 1 | 0.0038 | 0.0195 | 2 |
| 2 | 0.0038 | 0.0136 | 6 |
| 3 | 0.0037 | 0.0125 | 10 |
| 4 | 0.0021 | 0.0060 | 18 |
| 5 | 0.0024 | 0.0064 | 30 |
| 6 | 0.0015 | 0.0048 | 42 |
| | Vehicle-N % Dose | + Azone % Dose | |
| Total penetration | 0.118 | 0.390 | |
| Skin + surface | 89.935 | 82.937 | |
| Total recovery | 90.052 | 82.937 | |

After 48 hrs the epidermis was removed from the chamber and dissolved in 1 ml Soluene-350. Ten ml scintillation fluid was added and the samples counted. Duplicate counts were averaged and corrected for quenching by the external standard H count method. The results are shown in Table 4; the results suggest that penetration of TGF-$\beta$ is enhanced by Azone (5%).

Example 5

Penetration Through Human Skin

It is known that the stratum corneum of psoriatic skin is compromized. The relative penetration of TGF-$\beta$ into normal and psoriatic skin was evaluated as follows. Psoriatic skin was obtained from volunteers using a keratome at 0.4 mm. The sample of involved epidermis was placed on a Franz cell (see Figure 2) and perfused slowly from below with saline. A sample of normal human skin was used as a control. [$^{35}$S]-TGF-$\beta$ 3 x 10$^6$ cpm/$\mu$g was mixed with 4 $\mu$g/ml cold TGF-$\beta$ in phosphate buffered saline. Fifty $\mu$l of this solution containing 0.75 x 10$^6$cpm was placed on top of the skin section and diffusion of label into the lower chamber was monitored. Within the first 12 hrs 34% of the $^{35}$S-labeled protein diffused below the psoriatic lesion section. However, only 0.4% of the label diffused below normal skin, showing that more efficient penetration of TGF-$\beta$ is obtained with psoriatic skin.

## Table 5

### Penetration through human skin

**Involved Psoriatic Skin**

$$\frac{\text{CPM recovered}}{\text{CPM applied}} \; [^{35}\text{S}]\text{-TGF-}\beta$$

$$\frac{249,417}{722,958} = 33.8\%$$

**Normal Human Skin**

$$\frac{\text{CPM recovered}}{\text{CPM applied}} \; [^{125}\text{I}]\text{-TGF-}\beta$$

$$\frac{6,024}{1,430,000} = 0.4\%$$

Example 6

TGF-$\beta$ Recovery from Dermatologic Creams

To evaluate the ability of various nonionic emulsifying cream bases to preserve the activity of TGF-$\beta$, similar TGF-$\beta$1 was formulated into one of four cream vehicles, A, B, C and D. Formulas A, B and D comprised petrolatum, glycerin and dimethicone in a nonionic emulsifying cream base. The percentage of each of these components in the three formulations is shown in Table 6.

Table 6

| Amount of Petrolatum, Dimethicone AND Glycerin In Formulations A, B AND D | | | |
|---|---|---|---|
| Formulation | Petrolatum | Dimethicone | Glycerin |
| A | 30 | 1 | 5 |
| B | 6 | 3 | 5 |
| D | 40 | 0 | 0 |

Formula C is an oil and water (o/w) type, creamy white, emulsion base comprising the ingredients shown in Table 7.

Table 7

| Composition of Formulation C | |
|---|---|
| Ingredient | % of (w/w) |
| Water | 69.0 |
| Polypropylene glycol-15 | 8.0 |
| Cetyl esters | 5.0 |
| Cetyl stearyl alcohol | 4.0 |
| Glyceryl stearate | 3.0 |
| Sorbitol stearate | 3.0 |
| Sorbitol | 3.0 |
| Polysorbate 60 | 2.0 |
| Benzyl alcohol | 1.0 |
| 'Methylchloroisothiazolinone and isothiazolinone | 0.05% |

'Sold as Kathon; available from Rohm and Haas, Pa.

The formulations were prepared by mixing 0.2g of each cream with 20ml of PBS containing one or ten μg of TGF-β. If used, human serum albumin (10 μg 0.2g) was then added to the formulation. The pH of the cream was H.S. The cream was incubated overnight at room temperature, then extracted with 1ml of distilled water 0.2g of cream. The cream was centrifuged out of the clear supernatant assayed using the radioreceptor assay described in Science (1986) Bourne et al. :531-534 for TGF-β activity Formulation C, which did not contain dimethicone, petrolatum or glycerin, preserved the activity of TGF-β better than did those formulations containing these components. However, in the presence of human serum albumin (10 μg,0.2g), recovery of TGF-β was similar for formulations A and C.

It is evident from the above results that compositions comprising TGF-β have been provided which are capable of antagonizing proliferation of keratinocytes induced by IL-1, EGF and TGF-α. Thus the compositions may provide a means for treating hyperplastic dermatological disorders such as psoriasis.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now having been fully described, it will be apparent to one of ordinary skill in the art that many changes and modifications can be made thereto without departing from the spirit or scope of the appended claims.

**Claims**

1. A method for inhibiting proliferation of epidermal cells, said method comprising :
contacting said cells with a composition consisting essentially of at least one transforming growth factor-beta (TGF-β) or fragment thereof comprising at least eight consecutive amino acids of the complete sequence of said #TGF-β, wherein said composition is characterized as being capable of inhibiting proliferation of epidermal cells.

2. A method according to Claim 1, wherein said epidermal cells are keratinocytes.

3. A method according to Claim 1, wherein said transforming growth factor beta comprises TGF-β1 or TGF-β2.

4. A method for inhibiting cytokine-induced proliferation of keratinocytes, said method comprising:
contacting said cells with an inhibitor of said cytokine-induced proliferation, said inhibitor consisting essentially of a polypeptide comprising at least eight consecutive amino acids included in the complete sequence of a transforming growth factor-beta, wherein said polypeptide is characterized as being capable of inhibiting proliferation of keratinocytes.

5. A method according to Claim 4, wherein said cytokine is at least one of a TGF-α, interleukin I, and EGF.

6. A method for inhibiting proliferation of a plurality of hyperproliferating epidermal cells, said method comprising:

contacting said cells with a proliferation inhibiting amount of a polypeptide comprising TGFβ1 or TGFβ2 or a congener thereof.

7. A pharmaceutical composition comprising a cell proliferation inhibiting amount of at least one transforming growth factor-beta or a congener thereof in a physiologically acceptable carrier.

8. A composition according to Claim 7, wherein said physiologically acceptable carrier further comprises an epidermal penetration enhancer.

9. A composition according to Claim 8, wherein said enhancer comprises dimethyl sulfoxide at a concentration of from about 5 to about 50%.

10. A composition according to Claim 9, wherein said concentration is about 25%.

12

Fig. 1

SIMIAN TGF-β cDNA

```
Simian                    -261 AGGGGATCTGTGGCAGGTCGGAGA---AAGATC---CGTCTCCTGGTACCAGATCTCGCCCATCTAGGTT   -198
Human                          CTCC..C...CCA...A..CCCT.TTC....C.ACC.AC..T.............G..............

Simian   ATTTCCGTGGGATACTGAGACACCCCCGGTCCAAGCCTCCCCTCCACCACTGCGCCCTTCTCCCTGAGGA-CCTCAACTTTCCCTCGAGGCCCTCCTAC   -100
Human    ......................................................G....G.....................................

Simian   CTTTTCCCGGGGGACCCCCAGCCCCTGCAGGGGCGGGGCCTCCCCACCAAACTAGCCCTGTTCGCGCTCTCGGCAGTGCCGGGGGGCGCCGCCTCCCCC    -1
Human    .....G.....A............................................C..C.....................................

                                                                                                      Gly Pro
         Met Pro Pro Ser Gly Leu Arg Leu Leu Pro Leu Leu Leu Pro Leu Leu Trp Leu Leu Val Leu Thr Pro Ser Arg
Simian   ATG CCG CCC TCC GGG CTG CGG CTG CTG CCG CTG CTG CTA CCG CTG CTG TGG CTA CTG GTG CTG ACG CCT AGC CGG    75
Human    ... ... ... ... ... ... ... ... ... ... ... ... ... ... ... ... ... ... ... ... ... G.. .C.

                                              35                                  45          Ala
         Pro Ala Ala Gly Leu Ser Thr Cys Lys Thr Ile Asp Met Glu Leu Val Lys Arg Lys Arg Ile Glu Thr Ile Arg
Simian   CCG GCC GCA GGA CTA TCC ACC TGC AAG ACT ATC GAC ATG GAG CTG GTG AAG CGG AAG CGC ATC GAG ACC ATC CGC   150
Human    ... ... ... ... ... ... ... ... ... ... ... ... ... ... ... ... ... ... ... ... G.. ... ... ...

                                          60                                  70
         Gly Gln Ile Leu Ser Lys Leu Arg Leu Ala Ser Pro Pro Ser Gln Gly Glu Val Pro Pro Gly Pro Leu Pro Glu
Simian   GGC CAG ATC CTG TCC AAG CTG CGG CTC GCC AGC CCC CCG AGC CAG GGG GAG GTG CCG CCC GGC CCG CTG CCC GAG   225
Human    ... ... ... ... ... ... ... ... ... ... ... ... ... ... ... ... ... ... ... ... ... ... ... ...

                                          85                                  95
         Ala Val Leu Ala Leu Tyr Asn Ser Thr Arg Asp Arg Val Ala Gly Glu Ser Ala Glu Pro Glu Pro Glu Pro Glu
Simian   GCC GTG CTC GCC CTG TAC AAC AGC ACC CGC GAC CGG GTG GCC GGG GAG AGT GCA GAG CCG GAG CCC GAA CCG GAG   300
Human    ... ... ... ... ... ... ... ... ... ... ... ... ... ... ..A ..A ... ... ... ... ..G ..T ...

                                          110                                 120
         Ala Asp Tyr Tyr Ala Lys Glu Val Thr Arg Val Leu Met Val Glu Thr His Asn Glu Ile Tyr Asp Lys Phe Lys
Simian   GCC GAC TAC TAC GCC AAG GAG GTC ACC CGC GTG CTA ATG GTG GAA ACC CAC AAC GAA ATC TAT GAC AAG TTC AAG   375
Human    ... ... ... ... ... ... ... ... ... ... ... ... ... ... ... ... ... ... ... ... ... ... ... ...

                                          135                                 145
         Gln Ser Thr His Ser Ile Tyr Met Phe Phe Asn Thr Ser Glu Leu Arg Glu Ala Val Pro Glu Pro Val Leu Leu
Simian   CAG AGC ACA CAC AGC ATA TAT ATG TTC TTC AAC ACA TCA GAG CTC CGA GAA GCA GTA CCT GAA CCT GTG TTG CTC   450
Human    ... ..T ... ... ... ... ... ... ... ... ... ... ... ... ...G ... ... ..C ... ... ... ...

                                    Arg   160                                 170
         Ser Arg Ala Glu Leu Arg Leu Leu --- Arg Leu Lys Leu Lys Val Glu Gln His Val Glu Leu Tyr Gln Lys Tyr
Simian   TCC CGG GCA GAG CTG CGT CTG CTG --- AGG CTC AAG TTA AAA GTG GAG CAG CAT GTG GAG CTG TAC CAG AAA TAC   522
Human    ... ... ... ... ... ... ... ... AGG ... ... ... ... ..C ... ... ... ... ... ... ... ... ...

                                          185                        Asp         195
         Ser Asn Asn Ser Trp Arg Tyr Leu Ser Asn Arg Leu Leu Ala Pro Ser Asn Ser Pro Glu Trp Leu Ser Phe Asp
Simian   AGC AAC AAT TCC TGG CGA TAC CTC AGC AAC CGG CTG CTG GCG CCC AGC AAC TCG CCG GAG TGG TTG TCT TTT GAT   597
Human    ... ... ... ... ... ... ... ... ... ... ... ..A ... G.. ... ..A ... ... ..A ... ... ...

                                          210                                 220
         Val Thr Gly Val Val Arg Gln Trp Leu Ser Arg Gly Gly Glu Ile Glu Gly Phe Arg Leu Ser Ala His Cys Ser
Simian   GTC ACC GGA GTT GTG CGG CAG TGG TTG AGC CGC GGA GGG GAA ATT GAG GGC TTT CGC CTT AGC GCC CAC TGC TCC   672
Human    ... ... ... ... ... ... ... ... ... ..T ... ... ... ... ... ... ... ... ... ... ... ...

                         Arg                235
         Cys Asp Ser Lys Asp Asn Thr Leu Gln Val Asp Ile Asn Gly Phe Thr Thr Gly Arg Arg Gly Asp Leu Ala Thr
Simian   TGT GAC AGC AAA GAT AAC ACA CTG CAA GTG GAC ATC AAC GGG TTC ACT ACC GGC CGC CGA GGT GAC CTG GCC ACA   747
Human    ... ... ... .GG ... ... ... ... ... ... ... ... ... ... ... ... ... ... ... ... ... ..C

                                          260                                 270
         Ile His Gly Met Asn Arg Pro Phe Leu Leu Leu Met Ala Thr Pro Leu Glu Arg Ala Gln His Leu Gln Ser Ser
Simian   ATT CAT GGC ATG AAC CGG CCT TTC CTG CTT CTC ATG GCC ACC CCA CTG GAG AGG GCC CAA CAT CTG CAA AGC TCC   823
Human    ... ... ... ... ... ... ... ... ... ... ... ... ... ..G ... ... ... ... ..G ... ... ... ...

                             285                                 295
         Arg His Arg Arg Ala Leu Asp Thr Asn Tyr Cys Phe Ser Ser Thr Glu Lys Asn Cys Cys Val Arg Gln Leu Tyr
Simian   CGG CAC CGC CGA GCC CTG GAC ACC AAC TAC TGC TTC AGC TCC ACG GAG AAG AAC TGC TGC GTG CGG CAG CTG TAT   897
Human    ... ... ... ... ... ... ... ... ..T ... ... ... ... ... ... ... ... ... ... ... ... ..C

                             310                                 320
         Ile Asp Phe Arg Lys Asp Leu Gly Trp Lys Trp Ile His Glu Pro Lys Gly Tyr His Ala Asn Phe Cys Leu Gly
Simian   ATT GAC TTC CGC AAG GAC CTC GGC TGG AAG TGG ATC CAC GAG CCC AAG GGC TAC CAT GCC AAC TTC TGC CTG GGG   972
Human    ... ... ... ... ... ... ... ... ... ... ... ... ... ... ... ... ... ... ... ... ..C ...

                             335                                 345
         Pro Cys Pro Tyr Ile Trp Ser Leu Asp Thr Gln Tyr Ser Lys Val Leu Ala Leu Tyr Asn Gln His Asn Pro Gly
Simian   CCC TGT CCC TAC ATT TGG AGC CTG GAC ACG CAG TAC AGC AAG GTC CTG GCC CTG TAC AAC CAG CAT AAC CCG GGC   1047
Human    ... ..C ... ... ... ... ... ... ... ... ... ... ... ... ... ... ... ... ... ... ... ...

                             360                                 370
         Ala Ser Ala Ala Pro Cys Cys Val Pro Gln Ala Leu Glu Pro Leu Pro Ile Val Tyr Tyr Val Gly Arg Lys Pro
Simian   GCC TCG GCG GCG CCG TGC TGC GTG CCG CAG GCG CTG GAG CCA CTG CCC ATC GTG TAC TAC GTG GGC CGC AAG CCC   1122
Human    ... ... ... ... ... ... ... ... ... ..G ... ... ... ... ... ... ... ... ... ... ...

                             385
         Lys Val Glu Gln Leu Ser Asn Met Ile Val Arg Ser Cys Lys Cys Ser
Simian   AAG GTG GAG CAG CTG TCC AAC ATG ATC GTG CGC TCC TGC AAA TGC AGC TGA GGCCCCGCCCCGCCCCGCCCCACCCCGGCAG   1204
Human    ... ... ... ... ... ... ... ... ... ..G ... ... ... ... ... T...................G........

Simian   GCCCGGCCCCGCCCCACCCCACCCCCGCTGTCTTGGCCCTTGGGGGCTGTATTTAAGGACACCCGTGCCCCAAGCCCACCTGGGGCCCCATTAAAGA   1300
Human    ..........A....G....G.........C......A...........................................................
```

Fig. 2

Fig. 3

% Recovery (Bioactivity)

1ug TGF-b

10ug TGF-b

1ug TGF-b + 10ug HSA

Dermatalogic Creams (Samples A-D)

Figure 4